(19) Europäisches Patentamt
European Patent Office
Office européen des brevets

(11) EP 4 218 755 A1

(12) **EUROPEAN PATENT APPLICATION**
published in accordance with Art. 153(4) EPC

(43) Date of publication:
**02.08.2023 Bulletin 2023/31**

(21) Application number: **21889522.5**

(22) Date of filing: **02.11.2021**

(51) International Patent Classification (IPC):
***A61K 31/4155*** $^{(2006.01)}$ ***A61K 9/20*** $^{(2006.01)}$
***A61P 19/06*** $^{(2006.01)}$ ***A61P 9/04*** $^{(2006.01)}$
***A61P 9/12*** $^{(2006.01)}$ ***A61P 3/10*** $^{(2006.01)}$
***A61P 13/12*** $^{(2006.01)}$ ***A61P 29/00*** $^{(2006.01)}$
***A61P 19/02*** $^{(2006.01)}$ ***A61P 1/00*** $^{(2006.01)}$

(52) Cooperative Patent Classification (CPC):
**A61K 9/20; A61K 31/4155; A61P 1/00; A61P 3/10; A61P 9/04; A61P 9/12; A61P 13/12; A61P 19/02; A61P 19/06; A61P 29/00; C07D 403/04**

(86) International application number:
**PCT/KR2021/015708**

(87) International publication number:
**WO 2022/098057 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.11.2020 KR 20200145750**

(71) Applicant: **Lg Chem, Ltd.**
**Seoul 07336 (KR)**

(72) Inventors:
- **LEE, Seok Ju**
  **Daejeon 34122 (KR)**
- **PARK, Ah Byeol**
  **Daejeon 34122 (KR)**
- **JEONG, Hui Rak**
  **Daejeon 34122 (KR)**
- **HAM, Jinok**
  **Daejeon 34122 (KR)**
- **SHIN, Doosup**
  **Daejeon 34122 (KR)**

(74) Representative: **Hoffmann Eitle**
**Patent- und Rechtsanwälte PartmbB**
**Arabellastraße 30**
**81925 München (DE)**

(54) **METHOD FOR PREPARING CRYSTALLINE PARTICLES OF 1-(3-CYANO-1-ISOPROPYL-INDOLE-5-YL)PYRAZOLE-4-CARBOXYLIC ACID, AND PHARMACEUTICAL COMPOSITION COMPRISING SAME**

(57) The present invention relates to a pharmaceutical composition comprising crystalline particles comprising the compound of Formula 1 or a pharmaceutically acceptable salt thereof comprising the compound of Formula 2 below in an amount of 0.2 wt.% or less.

The crystalline particles according to the present invention, have a size, shape and distribution that improve uniformity and flowability as well as being optimized for input into the preparation process of the finished drug product, thereby increasing the content uniformity in the preparation process of the finished product and minimizing breakage during compressing into tablets, and thus can be used as a raw material pharmaceutical product suitable for the preparation process of the finished drug product.

【FIG. 1】

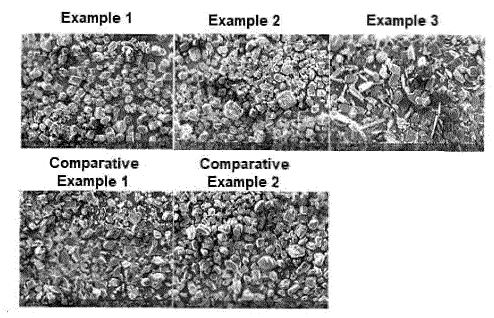


EP 4 218 755 A1

## Description

[Technical Field]

This application claims the benefit of priority based on Korean Patent Application No. 10-2020-0145750 filed on November 04, 2020, the entire contents of which are incorporated herein by reference.

The present invention relates to a pharmaceutical composition comprising crystalline particles of a compound of Formula 1 (1-(3-cyano-1-isopropyl-indol-5-yl)pyrazole-4-carboxylic acid) or a pharmaceutically acceptable salt thereof, comprising 0.2% by weight or less of a compound of Formula 2 below (1-(3-cyano-1-isopropyl-1H-indol-5-yl)-1H-pyrazole-4-methyl ester):

[Formula 1]

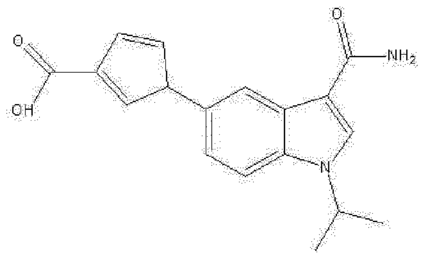

[Formula 2]

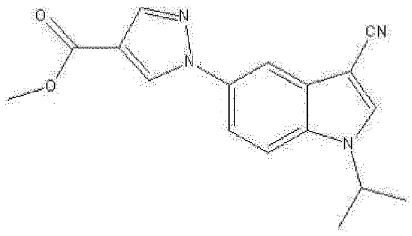

[Background Art]

Xanthine oxidase is an enzyme that converts hypoxanthine to xanthine and also the formed xanthine to uric acid. When there is too much uric acid in the body, it causes various diseases including gout disease, etc.

Gout disease refers to a condition in which uric acid crystals accumulate in the cartilage, ligaments, and surrounding tissues of the joint, causing severe inflammation and pain, and the incidence of gout disease has been steadily increasing over the past 40 years.

Therefore, substances that inhibit the activity of xanthine oxidase can effectively treat xanthine oxidase-related diseases, such as hyperuricacidemia, gout disease, heart failure, cardiovascular diseases, hypertension, diabetes, kidney diseases, inflammation, joint diseases and inflammatory bowel diseases.

On the other hand, as a substance inhibiting the activity of xanthine oxidase, Korean Patent No. 1751325 (Patent Document 1) provides a compound of Formula 1 (1-(3-cyano-1-isopropyl-indol-5-yl) pyrazole-4-carboxylic acid) and a method for preparing the compound, and Korean Patent No. 1424013 (Patent Document 2) provides various types of crystalline forms obtained by using various solvents and methods for preparing the same.

However, the crystalline particles of Formula 1 according to the conventional preparation method of Patent Document 1 have a problem of poor flowability, so there was a difficulty in the stable and reproducible preparation process of the finished product, and in the case of Patent Document 2, it relates to each crystalline form itself, and the flowability thereof has not been analyzed.

Therefore, there is a need for further development of the crystalline particles of Formula 1 with improved flowability, which are optimized for the size, shape, and distribution of the crystalline particles to be added to the preparation process of the finished product.

[Prior Art Document]

[Patent Document]

(Patent Document 1) Korean Patent No. 1751325 (2017.06.21.), Novel compounds effective as xanthine oxidase inhibitors, method for preparing the same, and pharmaceutical composition comprising the same

(Patent Document 2) Korean Patent No. 1424013 (2014.07.22.), 1-(3-cyano-1-isopropyl-indol-5-yl)pyrazole-4-carboxylic acid crystalline form and the producing method thereof

[Disclosure]

[Technical Problem]

Accordingly, the inventors of the present invention have conducted various studies to solve the above problems, and as a result, have confirmed that compounds of Formula 2 and Formula 3 may be generated during the manufacturing process of the compound of Formula 1 below, and that by adjusting the content of the compound of Formula 2 to a specific range, it is possible to obtain crystalline particles optimized for input into the preparation process of the finished drug product, thereby completing the present invention.

Accordingly, it is an object of the present invention to provide crystalline particles of the compound of Formula 1 or a pharmaceutically acceptable salt thereof, comprising 0.2 wt.% or less of the compound of Formula 2 optimized for input into the preparation process of the finished drug product, and a pharmaceutical composition including the same.

[Formula 1]

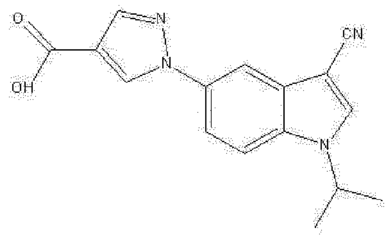

[Formula 2]

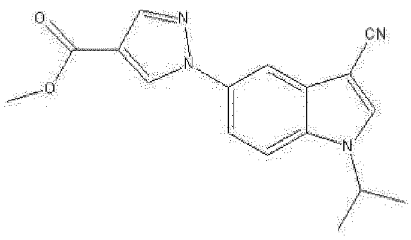

[Formula 3]

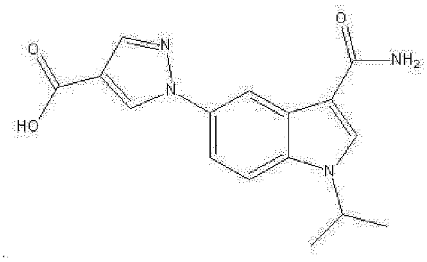

[Technical Solution]

The present invention provides crystalline particles of the compound of Formula 1 or a pharmaceutically acceptable salt thereof, comprising 0.2 wt.% or less of the compound of Formula 2 below.

[Formula 1]

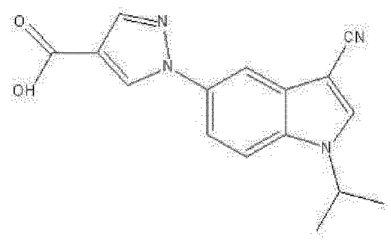

[Formula 2]

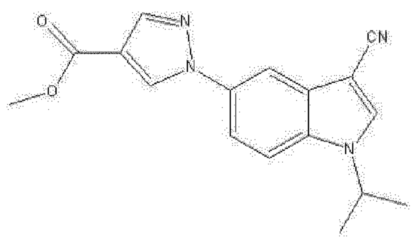

The crystalline particles of the present invention may further comprise the compound of Formula 3 below.

[Formula 3]

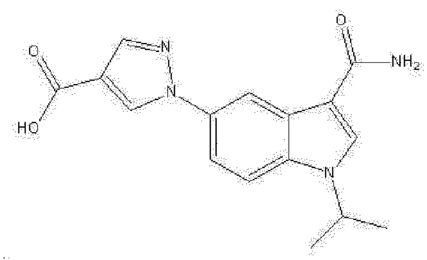

The present invention provides a method for preparing crystalline particles of Formula 1 comprising the compound of Formula 2 in an amount of 0.2 wt.% or less, and crystalline particles prepared by the above preparation method.

The Carr index of the crystalline particles of the present invention is 25 or less, 20 or less, 10 or less, or 7 or less.

The present invention provides a pharmaceutical composition for the treatment or prevention of xanthine oxidase-related diseases selected from the group consisting of hyperuricacidemia, gout disease, heart failure, cardiovascular diseases, hypertension, diabetes, renal diseases, inflammation, joint diseases and inflammatory bowel diseases, comprising crystalline particles of the compound of Formula 1 or a pharmaceutically acceptable salt thereof, comprising the compound of Formula 2 in an amount of 0.2 wt.% or less.

[Advantageous Effects]

The crystalline particles of the compound of Formula 1 or a pharmaceutically acceptable salt thereof comprising the compound of Formula 2 in an amount of 0.2 wt.% or less, according to the present invention, have a size, shape and distribution that improve uniformity and flowability as well as being optimized for input into the preparation process of the finished drug product, thereby increasing the content uniformity in the preparation process of the finished product and minimizing breakage during compressing into tablets, and thus can be used as a raw material pharmaceutical product suitable for the preparation process of the finished drug product.

[Description of Drawings]

FIG. 1 shows micrographs of crystalline particles prepared in Examples and Comparative Examples.

[Best Mode]

Hereinafter, the present invention will be described in detail with reference to the accompanying drawings so that those of ordinary skill in the art to which the present invention pertains can easily practice the present invention.

The terms and words used in the present specification and claims should not be construed as limited to ordinary or dictionary terms, and should be construed in a sense and concept consistent with the technical idea of the present invention, based on the principle that the inventor can properly define the concept of a term to describe his invention in the best way possible.

The terminology used herein is for the purpose of describing particular embodiments only and is not intended to be limiting of the present invention. The singular forms "a," "an," and "the" include plural referents unless the context clearly dictates otherwise. It is to be understood that the terms "comprise", or "have", etc., as used in the present specification, are intended to designate the presence of stated features, numbers, steps, operations, components, parts or combinations thereof, but not to preclude the possibility of the presence or addition of one or more other features, numbers, steps, operations, components, parts, or combinations thereof.

In the present invention, compound of Formula 1 is 1-(3-cyano-1-isopropyl-indol-5-yl)pyrazole-4-carboxylic acid, which is known to inhibit xanthine oxidase and prevent the deposition of uric acid in the body. In addition, the compound of Formula 1 can treat or prevent diseases selected from the group consisting of hyperuricacidemia, gout disease, heart failure, cardiovascular diseases, hypertension, diabetes, renal diseases, inflammation, joint diseases and inflammatory bowel diseases which are diseases related to the deposition of uric acid in the body.

[Formula 1]

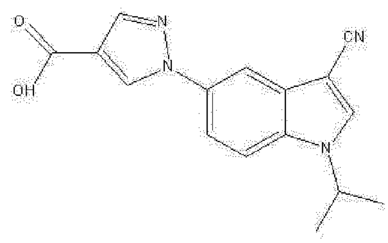

The compound of Formula 1 has a very low solubility characteristic in organic solvents and water due to the structural characteristics generated by the coupling reaction of pyrazole and indole, and thus has a characteristic that it is very difficult to control the size, shape and distribution of crystalline particles. In particular, the crystalline particles prepared by the preparation method of Patent Document 1, which is a conventional preparation method of the compound of Formula 1 have a problem of poor flowability, making it difficult for the preparation process of the finished drug product to be stable and reproducible. For the crystalline form compound of Patent Document 2, flowability was not analyzed,

and also there was a difficulty in manufacturing as a bulk raw material pharmaceutical product.

Accordingly, the researchers of the present invention have continued their research to prepare the crystalline particles of the above Formula 1 with good flowability, and as a result, have found that the compounds prepared by the conventional method include not only the compound of Formula 1, but also the compound of the following Formula 2 which is methyl-esterified at the C4 position of pyrazole of the compound of Formula 1 and the compound of the following Formula 3, which is an amide compound decomposed at the cyano group at the C3 position of the indole, and have confirmed that the shape and flowability of the crystalline particles vary depending on the amount of the compounds of Formula 2 and Formula 3.

[Formula 2]

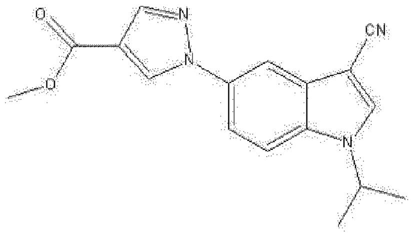

[Formula 3]

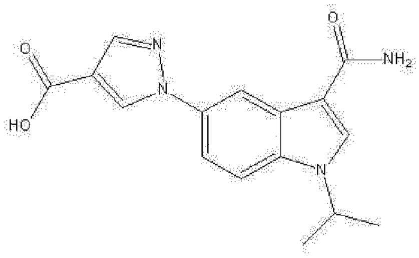

In the present invention, in order to analyze the effect of the compounds of Formula 2 and Formula 3 on on the generation of crystalline particles of the compound of Formula 1, which is an active material having inhibitory activity against xanthine oxidase, after preparing crystalline particles by adding the compound of Formula 2 or Formula 3 in an arbitrary amount to the compound of Formula 1 having a purity of 100%, Carr index and Hausner ratio related to flowability and cohesiveness of crystalline particles were analyzed by analyzing shape and particle size, tapped density, and apparent density of crystalline particles.

First, in the case of compound of Formula 2, as its content is increased, there are tendencies for the size of the crystalline particles to be non-uniform and for the plate-shaped crystalline particles to increase. However, in the case of compound of Formula 3, as its content is increased, since the shape of the crystalline particles is relatively close to that of a square hexahedron and their size is uniform, it can be seen that as the content of the compound of Formula 3 is increased, the shape and size of the crystalline particles become good (FIG. 1). Also, even in the case of particle size distribution, it can be seen that as the content of compound of Formula 2 is increased, the particle size distribution is not even, whereas as the content of Formula 3 is increased, the particle size distribution becomes even (Table 3).

The tapped density and bulk density were analyzed to analyze flowability and cohesiveness. tapped density means the changed volume when the container is tapped after packing the powder, which means that the voids between particles are reduced. In addition, the porosity can be confirmed by checking the changes in bulk density (apparent density) and tapped density. In general, the flowability and cohesiveness of the granular powder were evaluated using Carr index (CI) (Carr, 1965) and Hausner ratio (HR) (Hausner, 1967).

The table below shows the classification of the flowability of the granular powder according to the Carr index and Hausner ratio.

Table 1:

| Carr index(flowability) % | Flowability/cohesiveness | Hausner ratio(cohesiveness) |
|---|---|---|
| <10 | Excellent | 1.00~1.11 |

(continued)

| Carr index(flowability) % | Flowability/cohesiveness | Hausner ratio(cohesiveness) |
|---|---|---|
| 11~15 | Good | 1.12~1.18 |
| 16~20 | Fair | 1.19~1.25 |
| 21~25 | Passable | 1.26~1.34 |
| 26~31 | Poor | 1.35~1.45 |
| 32~38 | Very poor | 1.46~1.59 |
| >38 | Very, very poor | >1.6 |

Both bulk density and tapped density are properties related to the Carr index, which can affect a variety of formulations and in particular can affect the uniformity of the preparation. Specifically, the standard of the Carr index commonly used in raw materials in the pharmaceutical field is 20% or less, and a Carr index of 5 to 15% indicates excellent to good flowability. The Hausner ratio is the ratio of tapped density to bulk density and is an estimate of the friction between particles. A commonly used ratio is a ratio of 1.2 or less, which is an indication of acceptable friction, i.e. good flowability of the powder.

When the compound of Formula 2 is contained to the compound of Formula 1, if the compound of Formula 2 is contained in an amount of 0.246%, although the Carr index exceeds 20% and the Hausner ratio exceeds 1.25, these are values that can be used for pharmaceutical preparation. However, if the compound of Formula 2 is contained in an amount of 0.169%, the Carr index is 5.89% and the Hausner ratio is 1.06, showing very good results in both flowability and cohesiveness. However, if the compound of Formula 3 is contained, regardless of the good shape of the crystalline particles, the values of Carr index and Hausner ratio are higher than generally accepted values, and also it shows good results as its content is increased.

Accordingly, it can be seen that although both the compounds of Formula 2 and Formula 3 may be generated along with the compound of Formula 1 in the preparation process of the compound of Formula 1, as the content of the compound of Formula 2 is increased, the size and shape of the crystalline particles are irregular, and their flowability is deteriorated, whereas as the content of the compound of Formula 3 is increased, the size and shape of the crystalline particles become uniform, and their flowability becomes better.

Overally, in order to obtain crystalline particles having good flowability, it is preferable that the compound of Formula 1 comprises the compound of Formula 2 in an amount of 0.2 wt.% or less based on all of the crystalline particles. When comprised within the range of the above-mentioned content, the shape and size of the crystalline particles are good, the particle size distribution is even, and the flowability is good. However, when comprised outside the range of the above-mentioned content, since the shape and size of the crystalline particles become non-uniform, and the particle size distribution and flowability become poor, it cannot be used as a raw material pharmaceutical product.

A preparation method for preparing the desired crystalline particles is as follows.

The method comprises the steps of,

a) adding 1-(3-cyano-1-isopropyl-indol-5-yl)pyrazole-4-carboxylic acid ethyl ester, tetrahydrofuran, and methanol to the reactor, and then slowly adding 10 N NaOH;
b) further adding purified water and ethyl acetate;
c) crystallizing by dropwise addition of HCl; and
d) washing and drying the resulting crystals.

In addition, the reaction temperature of step a) in the preparation method is maintained at 21 to 27 °C.

In addition, the step of adding HCl dropwise in step c) can be divided and proceeded in two steps of,

c-1) firstly adding HCl dropwise until pH 5 to 6 to generate nuclei; and
c-2) secondly adding HCl dropwise to the time point of pH 2 to 3.

The content of the compound of Formula 2 of the crystalline particles prepared by the above preparation method is 0.2 wt.% or less, 0.1 wt.% or less, 0.05 wt.% or less.

The crystalline particles according to the present invention have a size, shape and distribution that improve uniformity and flowability as well as being optimized for input into the preparation process of the finished drug product, thereby increasing the content uniformity in the preparation process of the finished product and minimizing breakage during compressing into tablets, and thus can be used as a raw material pharmaceutical product suitable for the preparation process of the finished drug product.

The crystalline particles of the present invention can be administered as such to human patients as an active pharmaceutical ingredient (API), or administered with other active pharmaceutical ingredients as in combination therapy, or administered as a pharmaceutical composition in admixture with a suitable carrier or excipient.

The pharmaceutical composition of the present invention can be prepared in a known manner, for example, by means such as conventional mixing, dissolving, granulating, tableting, powdering, emulsifying, encapsulating, trapping or lyophilizing process.

Accordingly, the pharmaceutical composition according to the present invention can be prepared in a conventional manner using one or more pharmaceutically acceptable carriers which are intended to include excipients or adjuvants which facilitate processing of the active compound into a formulation that can be used pharmaceutically. Suitable formulations depend on the route of administration chosen. It is possible to use as appropriate any of the known techniques, the known carriers and excipients, and the means known in the art, for example, in Remingston's Pharmaceutical Sciences.

For example, in the present invention, the crystalline particles of the present invention may be formulated as an injectable preparation or an oral tablet, etc. according to the desired purpose, and preferably may be formulated as an oral tablet.

For injection, the components of the present invention may be formulated in a liquid solution, preferably in a pharmaceutically suitable buffer such as Hank's solution, Ringer's solution, or physiological saline. For administration by mucosal penetration, a suitable penetrating adjuvant is used in the formulation. Such penetrating adjuvants are generally known in the art.

For oral administration, the active compounds can be readily formulated by combining the active compounds with pharmaceutically acceptable carriers known in the art. Such carriers enable the compounds of the present invention to be formulated as tablets, powders, granules, dragees, capsules, liquids, gels, syrups, slurries, suspensions, and the like. Preferably, tablets, capsules, pills, powders and granules are used, and tablets are particularly useful. An oral tablet can be prepared, for example, as follows.

The oral preparation according to the present invention contains crystalline particles of the compound of Formula 1 comprising the compound of Formula 2 in an amount of 0.2% by weight or less, or a pharmaceutically acceptable salt thereof as API, and contains one or two or more excipients that may be selected from diluents, disintegrants, binders, glidants, stabilizers and lubricants.

For example, the diluent may be selected from the group consisting of microcrystalline cellulose, lactose monohydrate, lactose anhydride, lactose, starch, mannitol, carboxymethylcellulose, sorbitol, and combinations thereof, but is not limited thereto. The disintegrant may be selected from the group consisting of low-substituted hydroxypropyl cellulose, crospovidone, croscarmellose sodium, sodium starch glycolate, F-melt, and combinations thereof, but is not limited thereto. The binder may be selected from the group consisting of hydroxypropylcellulose, hydroxypropyl methylcellulose, hypromellose, polyvinylacetic acid, povidone, polyvinylpyrrolidone, copovidone, macrogol, sodium lauryl sulfate, light anhydrous silicic acid, synthetic aluminum silicate, silicate derivatives such as calcium silicate or magnesium metasilicate aluminate, phosphates such as calcium hydrogen phosphate, carbonates such as calcium carbonate, pre-gelatinized starch, gums such as acacia gum, gelatin, cellulose derivatives such as ethyl cellulose, and mixtures thereof, but is not limited thereto. The glidant may be selected from the group consisting of colloidal silicon dioxide, hydrated silicon dioxide, and combinations thereof, but is not limited thereto. The lubricant may be selected from the group consisting of magnesium stearate, silicon dioxide, talc, light anhydrous silicic acid, sodium stearyl fumarate, and combinations thereof, but is not limited thereto.

The content of API contained in the oral tablet may be about 20 to 70 wt.%, about 20 to 60 wt.%, about 20 to 50 wt.%, about 20 to 45 wt.%, about 30 to 70 wt.%, about 30 to 60 wt.%, or about 30 to 50 wt.%, about 30 to 45 wt.%, about 40 to 70 wt.%, about 40 to 60 wt.%, about 40 to 50 wt.%, about 40 to 45 wt.% based on the total weight of the oral tablet.

In addition, the API may be contained in an amount of, for example, about 50 mg to 500 mg, about 50 mg to 400 mg, about 50 mg to 300 mg, about 50 mg to 200 mg, about 50 mg to 100 mg, about 100 mg to 500 mg, about 100 mg to 400 mg, about 100 mg to 300 mg, about 100 mg to 200 mg, about 200 mg to 500 mg, about 200 mg to 400 mg, about 200 mg to 300 mg, about 300 mg to 500 mg, about 300 mg to 400 mg per unit dosage form.

In addition, the API may be contained in an amount of 50 mg, 100 mg, 150 mg, 200 mg, 300 mg, 400 mg or 500 mg per unit dosage form.

In addition, the compound of Formula 2 of the API may be contained in an amount of 0.2 wt.% or less based on the total content of the API.

The pharmaceutical composition according to the present invention contains crystalline particles of the compound of Formula 1 or a pharmaceutically acceptable salt thereof, comprising compound of Formula 2 in an amount of 0.2 wt.% or less, in an amount effective to achieve its intended purpose.

Specifically, a therapeutically effective amount means an amount of a compound effective to prolong the survival

of the subject being treated or to prevent, alleviate or ameliorate the symptoms of a disease. Determination of the therapeutically effective amount is within the ability of one of ordinary skill in the art, particularly in light of the detailed disclosure provided herein.

When formulated in a unit dosage form, it is preferable to contain as an active ingredient about 0.1 to 1,000 mg of crystalline particles of the compound of Formula 1 or a pharmaceutically acceptable salt thereof, comprising the compound of Formula 2 in an amount of 0.2 wt.% or less, per unit dosage form. The dosage is depending on the doctor's prescription based on factors such as the patient's weight, age, and the specific nature and symptoms of the disease. However, the dosage required for the treatment of adults is usually in the range of about 1 to 1000 mg per day, depending on the frequency and intensity of administration. When administered intramuscularly, intravenously or orally to adults, it will suffice usually for a total dose of about 1 to 500 mg per day as separate single doses. However, for some patients, a higher daily dose may be also desirable.

The present invention also provides a method for treating or preventing human xanthine oxidase-related diseases by using the crystalline particles of the compound of Formula 1 or a pharmaceutically acceptable salt thereof comprising the compound of Formula 2 in an amount of 0.2 wt.% or less, in a therapeutically effective amount.

The term "human xanthine oxidase-related disease" is a disease that can be treated or prevented by inhibiting human xanthine oxidase, which may be, for example, but is not limited to, hyperuricacidemia, gout disease, heart failure, cardiovascular diseases, hypertension, diabetes, diabete-related complications, renal diseases, inflammation, joint diseases and inflammatory bowel diseases. Examples of the diabete-related complications include hyperlipidemia, arteriosclerosis, obesity, hypertension, retinopathy, renal failure, etc. (Circulation Research, 2006, 98, 169-171; Hypertension 2003, 41, 1183-90).

The term "treatment" means stopping or delaying the progression of a disease, when used for a subject showing symptoms of the onset of a disease, and the term "prevention" means stopping or delaying the symptoms of the onset of a disease, when used for a subject that does not show symptoms of the onset of a disease but is at high risk of developing such a disease.

The present invention will be described in more detail based on the following Examples and Experimental Examples. However, these Examples and Experimental Examples are only for helping the understanding of the present invention, and the scope of the present invention is not limited to these Examples and Experimental Examples in any sense.

## Example

### Synthesis Example 1: Synthesis of compound of Formula 1

### Synthesis Example 1-1: Synthesis of 1-(3-cyano-1H-indol-5-yl)pyrazole-4-carboxylic acid ethyl ester

The title compound was obtained through the following procedures (1), (2), and (3).

#### (1) Synthesis of 1-(3-formyl-1H-indol-5-yl)pyrazole-4-carboxylic acid ethyl ester

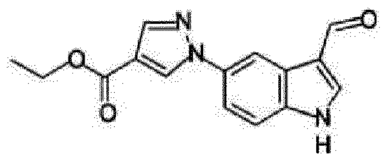

Oxalyl chloride (0.56 mℓ) was added to 50 mℓ of anhydrous dichloromethane, and N,N-dimethylformamide (0.51 mi) was added at 0°C, followed by stirring at 0°C for 30 minutes. To this reaction solution, a mixture of compound 1-(1H-indol-5-yl)pyrazole-4-carboxylic acid ethyl ester (1.40 g) and 50 mℓ of dichloromethane was added, and stirred at room temperature under reflux for 1 hour, and then the solvent was removed. 100 mℓ of tetrahydrofuran and 100 ml of a 20% aqueous ammonium acetate solution were added, and the mixture was stirred under reflux while heating for 30 minutes. After completion of the reaction, the reaction solution was cooled, ethyl acetate was added, washed with an aqueous sodium hydrogen carbonate solution, and then the organic layer was dried over anhydrous magnesium sulfate and concentrated under reduced pressure to obtain the title compound.

**(2) Synthesis of 1-[3-[(E,Z)-hydroxyiminomethyl]-1H-indol-5-yl]pyrazole-4-carboxylic acid ethyl ester**

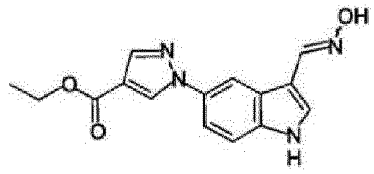

1-(3-formyl-1H-indol-5-yl)pyrazole-4-carboxylic acid ethyl ester obtained in step (1) was dissolved in 150 mℓ of pyridine, and hydroxyammonium chloride (499 mg) was added thereto. The mixture was stirred under reflux while heating for 5 hours. After completion of the reaction, the solvent was concentrated under reduced pressure and filtered through silica gel using acetone as a solvent to obtain the title compound.

**(3) Synthesis of 1-(3-cyano-1H-indol-5-yl)pyrazole-4-carboxylic acid ethyl ester**

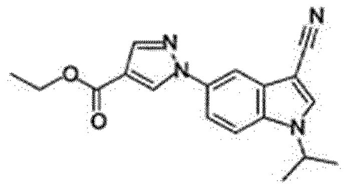

1-[3-[(E,Z)-hydroxyiminomethyl]-1H-indol-5-yl]pyrazole-4-carboxylic acid ethyl ester obtained in step (2) was dissolved in 94 mℓ of anhydrous tetrahydrofuran, and di(imidazol-1-yl)methanethione (90%, 2.79 g) was added thereto, and then stirred at room temperature for 2 hours. After completion of the reaction, the reaction solution was concentrated under reduced pressure, and the resulting solid compound was separated by column chromatography to obtain 1.32 g (86% yield) of the title compound.

**Synthesis Example 1-2: Synthesis of 1-(3-cyano-1-isopropyl-indol-5-yl)pyrazole-4-carboxylic acid ethyl ester**

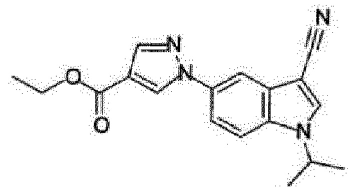

1-(3-cyano-1H-indol-5-yl)pyrazole-4-carboxylic acid ethyl ester (13.84g) obtained in Preparation Example 1-1 was dissolved in 200 mℓ of acetonitrile. Cesium carbonate (32.17 g) and 2-iodopropane (19.7 mℓ) were added thereto, and the mixture was stirred under reflux while heating for 5 hours. After completion of the reaction, the reaction solution was concentrated under reduced pressure, and the resulting solid compound was separated by column chromatography to obtain 13.87 g (87% yield) of the title compound.

**Synthesis Example 1-3: Synthesis of 1-(3-cyano-1-isopropyl-indol-5-yl)pyrazole-4-carboxylic acid (Formula 1) and preparation of crystalline particles**

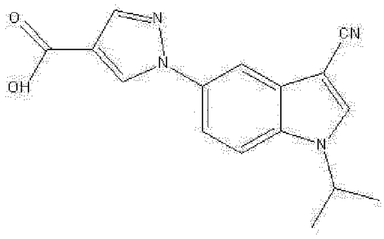

1-(3-cyano-1-isopropyl-indol-5-yl)pyrazole-4-carboxylic acid ethyl ester (16.09 kg) obtained in Synthesis Example 1-2, tetrahydrofuran (22.21 kg), and methanol (19.7 kg) were added to the reactor, and 10 N NaOH (33.11 kg) was slowly added at about 8 °C to allow the reaction to proceed. After completion of the reaction, purified water (24.95 L), conc. HCl (25.99 kg) was slowly added dropwise while maintaining 0 to 10 °C and aged, and then filtration was carried out. The filtered solid was washed with purified water and then vacuum dried to obtain a final product (14.83 kg) in the form of crystalline particles of 1-(3-cyano-1-isopropyl-indol-5-yl)pyrazole-4-carboxylic acid (Formula 1). As a result of component analysis of the final product, it was confirmed that the product mainly includes the compound of Formula 1 as shown below, but includes some of the compounds of Formula 2 and Formula 3 as shown below, and in particular, the content of the compound of Formula 2 was measured to be 0.24%.

[Formula 1]

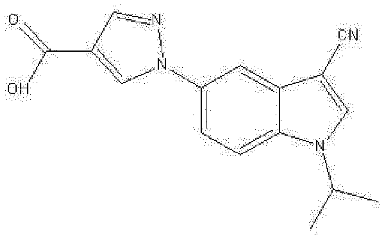

[Formula 2]

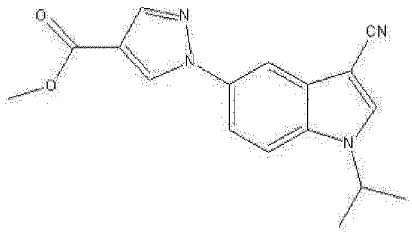

[Formula 3]

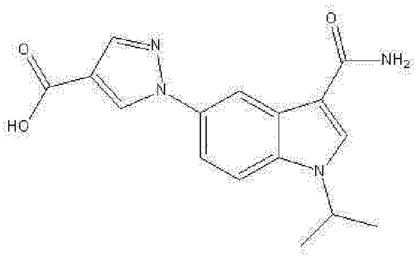

**Preparation Example 1: Preparation of crystalline particles of Formula 1 comprising various concentrations of an additive compound (compound of Formula 2)**

In order to analyze the role of Formula 2 comprised in the crystalline particles of the compound of Formula 1 prepared in Synthesis Example 1-3 above, the compound of Formula 1 was mixed with a specific amount of compound of Formula 2 by the following method to prepare crystalline particles (Examples 1 to 3).

After mixing the compound of Formula 1 and the compound of Formula 2 at the content ratio shown in Table 2, 90 mℓ of acetone and 9.9 mℓ of EtOAc were added, and then 105 mℓ of 1N NaOH was slowly added dropwise for 1 hour and stirred to remove solid impurities. 0.15 mℓ of 6N HCl was added to the filtrate to lower the pH to pH=7-8, and then heated to 50±5°C, and 16.2 mℓ of 6N HCl was slowly added while maintaining this temperature. After the dropwise addition was completed, pH=5±0.5 of the reaction mixture was confirmed, and cooling was started to lower the temperature of the reaction mixture to 25 °C, followed by filtration. The filtered solid was washed with purified water and then dried to obtain crystalline particles which is the target compound. As a result of measuring the content of the compound of Formula 2 in the obtained crystalline particles by HPLC, it was confirmed as 0.169% (Example 1), 0.246% (Example 2) and 1.646% (Example 3).

**Preparation Example 2: Preparation of crystalline particles of Formula 1 comprising various concentrations of an additive compound (compound of Formula 3)**

In order to analyze the role of Formula 3 comprised in the crystalline particles of the compound of Formula 1 prepared in Synthesis Example 1-3 above, the compound of Formula 1 was mixed with a specific amount of compound of Formula 3 by the following method to prepare crystalline particles (Comparative Examples 1 and 2).

40 g of compound of Formula 1, 1.25 g of compound of Formula 3, 120 mℓ of acetone, and 140 mℓ of 1N NaOH were slowly added dropwise over 1 hour. After the mixture was stirred, solid impurities were removed. 0.2 mℓ of 6N HCl was added to the filtrate to lower the pH to pH=7-8, and then heated to 50±5°C, and 21.6 mℓ of 6N HCl was slowly added while maintaining this temperature. After the dropwise addition was completed, pH=5±0.5 of the reaction mixture was confirmed, and cooling was started to lower the temperature of the reaction mixture to 25 °C, followed by filtration. The filtered solid was washed with purified water and then dried to obtain crystalline particles which is the target compound. As a result of measuring the content of the compound of Formula 3 in the obtained crystalline particles by HPLC, it was confirmed as 0.153% (Comparative Example 1).

25 g of compound of Formula 1, 35 g of compound of Formula 3, 90 mℓ of acetone, 9.9 mℓ of EtOAc, and 105 mℓ of 1N NaOH were slowly added dropwise over 1 hour. After the mixture was stirred, solid impurities were removed. 0.15 mℓ of 6N HCl was added to the filtrate to lower the pH to pH=7-8, and then heated to 50±5°C, and 16.2 mℓ of 6N HCl was slowly added while maintaining this temperature. After the dropwise addition was completed, pH=5±0.5 of the reaction mixture was confirmed, and cooling was started to lower the temperature of the reaction mixture to 25 °C, followed by filtration. The filtered solid was washed with purified water and then dried to obtain crystalline particles which is the target compound. As a result of measuring the content of the compound of Formula 3 in the obtained crystalline particles by HPLC, it was confirmed as 1.243% (Comparative Example 2).

Table 2 below shows the content of the additive compound comprised in the crystalline particles prepared in each Example and Comparative Example.

Table 2:

| Note | | Example 1 | Example 2 | Example 3 | Comparative Example 1 | Comparative Example 2 |
|---|---|---|---|---|---|---|
| Compound of Formula 1 | | 29.95g | 29.925g | 29.5g | 40g | 25g |
| Additive compound | Formula 2 | 0.05g | 0.075g | 0.5g | - | - |
| | Formula 3 | - | - | - | 1.25g | 5g |
| Content of additive compound measured after purification process | | 0.169% | 0.246% | 1.6460 | 0.153% | 1.243% |

**Experimental Example 1. Analysis of the characteristics of each crystalline particle (Analysis of shape of crystalline particles)**

For the crystalline particles prepared in Examples and Comparative Examples, the shape of the particles was observed and measured with a scanning electron microscope (SEM).

As a result of the analysis, as shown in FIG. 1, the particles in the crystalline form of Examples 1 to 3 have a square hexahedral shape as the content of the compound of Formula 2 is increased, but there are tendencies for the size of the particles to be non-uniform (Example 2), and for non-uniform plate-shaped particles and fine powder to increase (Example 3) . Therefore, it can be seen that even if the compound of Formula 1 comprises 0.246% of the compound of Formula 2, some non-uniform particles are comprised, but elatively uniform square hexahedron shapes are shown.

However, Comparative Examples 1 and 2 show different aspects from Examples 1 to 3. The crystalline particles in Comparative Example 1 are not only non-uniform in shape, but also produce a lot of fine powder, so it is difficult to put them into the preparation process of the finished product. However, it can be seen that in the case of Comparative Example 2, the shape of the crystalline particles is relatively close to the shape of a square hexahedron, and the size of the particles is uniform, and in the case of the compound of Formula 3, as its content is increased, the shape and size of the crystalline particles become better.

Therefore, unlike Chemical Formula 2, since it can be seen that unlike the compound of Formula 2, the compound of Formula 3 shows crystalline particles having a uniform square hexahedron shape that can be put into the preparation process of the finished product as its content is increased, it can be seen that the compounds of Formulas 2 and 3 produced in the process of preparing the compound of Formula 1 have completely different properties from each other.

**Experimental Example 2. Analysis of the characteristics** of **crystalline particles (Analysis of particle size distribution)**

The volume average particle distribution and particle size distribution of the crystalline particles prepared in Preparation Examples, Examples and Comparative Examples were measured by a wet method using a laser diffraction particle size analyzer.

It can be said that the particle size distribution is better as the values of DV10/DV50 and DV50/DV90 used as indicators are larger. Here, DV10, DV50, and DV90 mean particle diameters of particles corresponding to 10%, 50%, and 90% of the total number of particles, respectively, when the measured particles are arranged in order from smallest to largest in particle size.

As shown in Table 1 below, it can be seen that yhe crystalline particles of Examples 1 and 2 have a relatively uniform particle size distribution compared to Comparative Examples, but the particle size of the particles becomes smaller. However, it can be seen that Example 3 and Comparative Examples 1 and 2 have uneven particle size distribution.

Table 3:

| | Size Classes ($\mu$m) | | | $DV_{10}/DV_{50}$ | $DV_{50}/DV_{90}$ |
|---|---|---|---|---|---|
| | DV (10) | DV (50) | DV (90) | | |
| Example 1 | 107 | 172 | 273 | 0.622 | 0.63 |
| Example 2 | 87.4 | 142 | 224 | 0.615 | 0.633 |
| Example 3 | 86.5 | 181 | 321 | 0.478 | 0.564 |
| Comparative Example 1 | 38 | 141 | 251 | 0.270 | 0.561 |
| Comparative Example 2 | 87.6 | 151 | 245 | 0.58 | 0.616 |

**Experimental Example 3. Analysis of bulk density and tapped density of crystalline particles (Analysis of flowability)**

The bulk density and tapped density of crystalline particles prepared according to Preparation Examples, Examples and Comparative Examples were measured. The bulk density is the volume when about 50 g of granular powder is put into the measuring cylinder, and the tapped density is the volume when there is no further change in volume after lightly tapping the measuring cylinder on the floor at a constant height 100 times. The bulk density refers to the volume occupied by a certain mass of powder, which refers to the sum of the volume of the powder and the volume of voids between particles as the total volume.

The flowability and cohesiveness of the granular powder were calculated by the Carr index of Equation (3) and the Hausner ratio of Equation (4) using the bulk density and tapped density measured above according to the method of Jinapong et al (2008).

Equation (3): $$CI = \frac{\rho_{tapped} - \rho_{bulk}}{\rho_{tapped}} X\ 100$$

Equation (4): $$HR = \frac{\rho_{tapped}}{\rho_{bulk}} X\ 100$$

Table 4:

| Note | Bulk density (g/mℓ) | Tapped density (g/mℓ) | Carr index (%) | Hausner ratio |
|---|---|---|---|---|
| Example1 | 0.76 | 0.81 | 5.89 | 1.06 |
| Example 2 | 0.62 | 0.78 | 21.21 | 1.27 |
| Example 3 | 0.45 | 0.64 | 29.74 | 1.42 |
| Comparative Example 1 | 0.45 | 0.71 | 35.58 | 1.56 |
| Comparative Example 2 | 0.64 | 0.82 | 21.83 | 1.28 |

Carr index is a measure of the compressibility of a powder and is defined as a percentage of (tapped density - bulk density)/tapped density. As the index is increased, the powder becomes more compactible and less flowable. The Husner ratio is the ratio of tapped density to bulk density and is an estimate of the friction between particles.

The crystalline particles of Example 1 have a Carr index of 5.89%, and a Hausner ratio of 1.06, and show very good results in both flowability and cohesiveness (Excellent), and the values of Example 2 are not as good as those of Example 1, but overally they have acceptable flowability and cohesiveness in a pharmaceutical formulation. However, although the crystalline particles of Example 3 showed good particle size distribution as in Example 1 in the particle size distribution analyzed above, since they have a Carr index of about 30% and a Hausner ratio of 1.42, they have characteristics that cannot be used in a general formulation.

The crystalline particles of Comparative Example 1 have a Carr index of 35.58% and a Hausner ratio of 1.56, which have inferior effects (poor flowability and high cohesiveness characteristics) than the crystalline particles of Example 3, but the crystalline particles of Comparative Example 2 have improved values compared to those of Comparative Example 1, which are similar to those of Example 2 above.

Therefore, in order to obtain crystalline particles including the compound of Formula 1 having good flowability, the compound of Formula 2 may be comprised in an amount of 0.246%, and when the content is 0.2% or less, it is possible to obtain crystalline particles with very good flowability.

**Synthesis Example 1-4: Synthesis of improved 1-(3-cyano-1-isopropyl-indol-5-yl)pyrazole-4-carboxylic acid (Formula 1) and preparation of crystalline particles**

Since it was confirmed from the above Experimental Examples that in the crystalline particles of the compound of Formula 1, as the compound of Formula 2 is incorporated in a smaller amount, especially in an amount of 0.2% or less, crystalline particles having uniformity and excellent flowability can be obtained. Therefore, by reflecting this, the preparation method of Formula 1 comprising the compound of Formula 2 in an amount of 0.2% or less was derived.

1-(3-cyano-1-isopropyl-indol-5-yl)pyrazole-4-carboxylic acid ethyl ester (312 kg) obtained in Synthesis Example 1-2, tetrahydrofuran (554 kg) and methanol (624 L) were added to the reactor, and then 10 N NaOH (386 kg) was slowly added. Since the reaction is an exothermic reaction, it was added for about 1 hour, while taking care not to exceed the internal temperature of 27 °C. After completion of the dropwise addition, the reaction was allowed to proceed in the range of 21 to 27 °C. After completion of the reaction, purified water (624 L) and ethyl acetate (197 kg) were added, and 3N HCl (1048 kg) was slowly added dropwise while maintaining 30 to 35 °C. 3N HCl was divided into first and second dropwise additions, and the first dropwise addition was carried out for the 1st nucleation of the solid until the time point at which pH = 5 to 6. The reaction mixture in which a solid was produced was stirred for 30 minutes, and then the second dropwise addition was carried out until the time point at which pH = 2 to 3. After completion of the dropwise addition, the mixture was cooled to room temperature, aged at this temperature for 30 minutes, and then filtered. The filtered solid was washed with purified water (624 L) and dried under nitrogen and vacuum to obtain a final compound (273.2 kg)

comprising 1-(3-cyano-1-isopropyl-indol-5-yl)pyrazole-4-carboxylic acid. In particular, the content of the compound of Formula 2 was measured to be 0.02 wt.%.

In addition, it was confirmed that the particle size distribution and flowability of the final product were very good.

Accordingly, it was confirmed that in the method of Synthesis Example 1-3, which is the existing manufacturing method of the compound of Formula 1, when the temperature of the process of mixing and reacting 1-(3-cyano-1-isopropyl-indol-5-yl)pyrazole-4-carboxylic acid ethyl ester, tetrahydrofuran, methanol and 10 N NaOH is set to 27°C, ethyl acetate is added together when adding purified water after the reaction, and the dropwise addition of HCl is divided into two steps, thus the content of the compound of Formula 2 may be adjusted to 0.2 wt.% or less.

**Experimental Example 4: Method for preparing an oral tablet comprising the compound of Formula 1 or a pharmaceutically acceptable salt thereof as an** active **pharmaceutical ingredient (API)**

In order to prepare an oral tablet comprising the compound of Formula 1 comprising partially the compound of Formula 2 as an API, the excipients listed in the table below were mixed with the API, and then oral tablets were prepared using a tablet machine.

Table 5:

| Function | Ingredient | Preparation Example 3 | Preparation Example 4 | Preparation Example 5 | Preparation Example 6 |
|---|---|---|---|---|---|
| API | Crystalline particles of Synthesis Examples 1-4 | 45.5% | 45.5% | 45.5% | 45.5% |
| Diluent | Microcrystal line cellulose | 47.6% | 43.6% | 43.6% | 43.6% |
| | Starch | - | - | - | - |
| | Lactose anhydride | - | - | - | - |
| Disintegra ting | Crospovidone | 4.4% | 4.5% | - | - |
| | Croscarmello se sodium | - | - | 4.5% | - |
| | Sodium starch glycolate | - | - | - | 4.50 |
| Binder | Copovidone | - | 4.5% | - | - |
| | Povidone | - | - | 4.50 | - |
| | Hydroxypropy 1 cellulose | - | - | - | 4.5% |
| Glidant | Colloidal silicon dioxide | 0.50 | 1% | 1% | 1% |
| Lubricant | Sodium stearyl fumarate | 20 | - | 0.8% | - |
| | Magnesium stearate | - | 0.80 | - | 0.8% |

## Claims

1. A pharmaceutical composition for the treatment or prevention of xanthine oxidase-related diseases selected from the group consisting of hyperuricacidemia, gout disease, heart failure, cardiovascular diseases, hypertension, diabetes, renal diseases, inflammation, joint diseases, and inflammatory bowel diseases, comprising crystalline particles of the compound of Formula 1 below or a pharmaceutically acceptable salt thereof comprising the compound of Formula 2 below in an amount of 0.2 wt.% or less; and a pharmaceutically acceptable excipient:

[Formula 1]

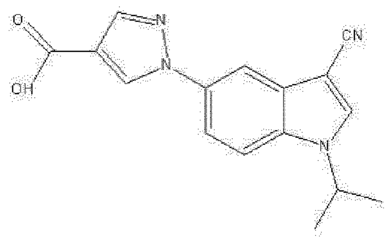

[Formula 2]

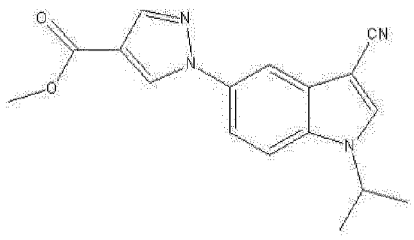

.

2. The pharmaceutical composition according to claim 1, wherein the content of the crystalline particles is 20 to 70 wt.% based on the total 100 wt.% of the pharmaceutical composition.

3. The pharmaceutical composition according to claim 2, wherein the content of the crystalline particles is 30 to 60 wt.% based on the total 100 wt.% of the pharmaceutical composition.

4. The pharmaceutical composition according to claim 3, wherein the content of the crystalline particles is 40 to 50 wt.% based on the total 100 wt.% of the pharmaceutical composition.

5. The pharmaceutical composition according to claim 1, wherein the following compound of Formula 3 is further comprised.

[Formula 3]

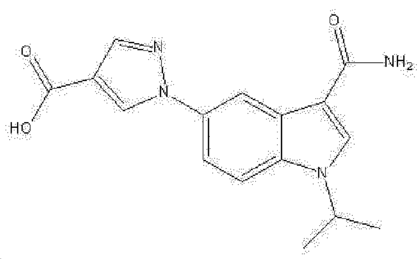

.

6. A method for preparing crystalline particles of Formula 1 comprising the compound of Formula 2 in an amount of 0.2 wt.% or less, which comprises the steps of,

a) mixing 1-(3-cyano-1-isopropyl-indol-5-yl)pyrazole-4-carboxylic acid ethyl ester, tetrahydrofuran, and methanol to the reactor, and then slowly adding NaOH and reacting it;
b) adding purified water and ethyl acetate;
c) crystallizing by dropwise addition of HCl; and
d) washing and drying the resulting crystals.

7. The method for preparing the crystalline particles according to claim 6, wherein in step a), the reaction temperature

is maintained at 21 to 27°C.

**8.** The method for preparing the crystalline particles according to claim 7, wherein step c) comprises two steps of,

c-1) firstly adding HCl dropwise until pH 5 to 6 to generate nuclei; and
c-2) secondly adding HCl dropwise until the time point of pH 2 to 3.

**9.** Crystalline particles prepared by the method of any one of claims 6 to 8.

**10.** The crystalline particles according to claim 9, wherein the crystalline particles contain 0.2 wt.% or less of the compound of Formula 2.

**11.** An oral tablet comprising the crystalline particles of claim 10 as an active pharmaceutical ingredient (API), wherein the content of the API is 20 to 70 wt.% based on the total 100 wt.% of the tablet.

**12.** The oral tablet according to claim 11, wherein content of the API is 30 to 60 wt.% based on the total 100 wt.% of the tablet.

**13.** The oral tablet according to claim 12, wherein content of the API is 40 to 50 wt.% based on the total 100 wt.% of the tablet.

【FIG. 1】

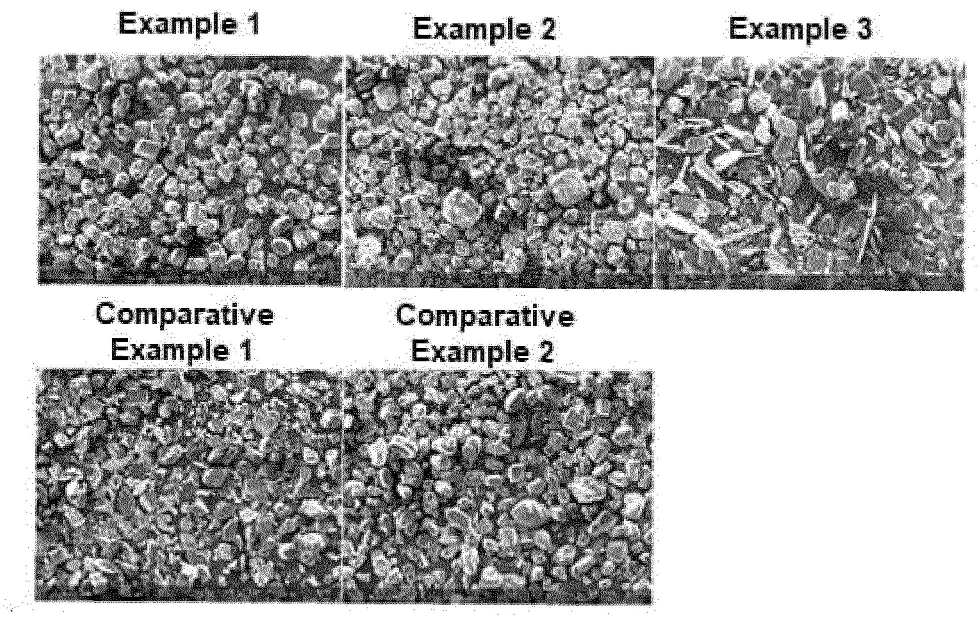
Example 1
Example 2
Example 3
Comparative Example 1
Comparative Example 2

**INTERNATIONAL SEARCH REPORT**

International application No. **PCT/KR2021/015708**

**A. CLASSIFICATION OF SUBJECT MATTER**

**A61K 31/4155**(2006.01)i; **A61K 9/20**(2006.01)i; **A61P 19/06**(2006.01)i; **A61P 9/04**(2006.01)i; **A61P 9/12**(2006.01)i; **A61P 3/10**(2006.01)i; **A61P 13/12**(2006.01)i; **A61P 29/00**(2006.01)i; **A61P 19/02**(2006.01)i; **A61P 1/00**(2006.01)i

According to International Patent Classification (IPC) or to both national classification and IPC

**B. FIELDS SEARCHED**

Minimum documentation searched (classification system followed by classification symbols)

A61K 31/4155(2006.01); A61K 31/404(2006.01); A61K 31/435(2006.01); C07D 209/42(2006.01); C07D 401/14(2006.01); C07D 403/04(2006.01); C07D 417/04(2006.01); C07D 491/056(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 1-(3-시아노-1-아이소프로필-인돌-5-일)피라졸-4-카르복실산(1-(3-cyano-1-isopropyl-indol-5-yl)pyrazole-4-carboxylic acid), 결정성 입자(crystalline particles), 잔틴옥시다아제(xanthine oxidase), 고요산혈증(hyperuricemia), 통풍(gout)

**C. DOCUMENTS CONSIDERED TO BE RELEVANT**

| Category* | Citation of document, with indication, where appropriate, of the relevant passages | Relevant to claim No. |
|---|---|---|
| DX<br>DA | KR 10-1424013 B1 (LG LIFE SCIENCES LTD.) 18 August 2014 (2014-08-18)<br>See claims 1-11; paragraphs [0019]-[0024], and example 1. | 1-4,6-13<br>5 |
| DX | KR 10-1751325 B1 (LG CHEM, LTD.) 27 June 2017 (2017-06-27)<br>See claims 1-3 and 7-8; and paragraphs [0195]-[0198] and [0479]-[0482]. | 1-4,6-13 |
| A | KR 10-2009-0128488 A (KISSEI PHARMACEUTICAL CO., LTD.) 15 December 2009 (2009-12-15)<br>See entire document. | 1-13 |
| A | WO 97-27186 A1 (MERCK PATENT GESELLSCHAFT MIT BESCHRAENKTER HAFTUNG) 31 July 1997 (1997-07-31)<br>See entire document. | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 February 2022** | **15 February 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
PCT/KR2021/015708

C. DOCUMENTS CONSIDERED TO BE RELEVANT

| Category* | Citation of document, with indication, where appropriate, of the relevant passages | Relevant to claim No. |
|---|---|---|
| A | WO 2008-126898 A1 (KISSEI PHARMACEUTICAL CO., LTD.) 23 October 2008 (2008-10-23)<br>See entire document. | 1-13 |

Form PCT/ISA/210 (second sheet) (July 2019)

**INTERNATIONAL SEARCH REPORT**
**Information on patent family members**

International application No.
**PCT/KR2021/015708**

| Patent document cited in search report | Publication date (day/month/year) | Patent family member(s) | Publication date (day/month/year) |
|---|---|---|---|
| KR 10-1424013 B1 | 18 August 2014 | CN 103459381 A | 18 December 2013 |
| | | CN 103459381 B | 09 December 2015 |
| | | JP 2014-510133 A | 24 April 2014 |
| | | JP 6008937 B2 | 19 October 2016 |
| | | KR 10-2012-0114174 A | 16 October 2012 |
| | | TW 201245182 A | 16 November 2012 |
| | | TW I548630 B | 11 September 2016 |
| | | WO 2012-138147 A2 | 11 October 2012 |
| | | WO 2012-138147 A3 | 29 November 2012 |
| KR 10-1751325 B1 | 27 June 2017 | AP 3346 A | 31 July 2015 |
| | | AR 078504 A1 | 09 November 2011 |
| | | AU 2010-304091 A1 | 19 April 2012 |
| | | AU 2010-304091 B2 | 09 June 2016 |
| | | BR 112012007828 A2 | 08 March 2016 |
| | | BR 112012007828 B1 | 12 January 2021 |
| | | BR 112012007828 B8 | 25 May 2021 |
| | | CA 2774133 A1 | 14 April 2011 |
| | | CA 2774133 C | 25 June 2019 |
| | | CL 2012000738 A1 | 14 September 2012 |
| | | CN 102574839 A | 11 July 2012 |
| | | CN 102574839 B | 25 November 2015 |
| | | CO 6430501 A2 | 30 April 2012 |
| | | EA 021025 B1 | 31 March 2015 |
| | | EA 201270520 A1 | 30 October 2012 |
| | | EC SP12011793 A | 30 May 2012 |
| | | EP 2467378 A2 | 27 June 2012 |
| | | EP 2467378 A4 | 24 April 2013 |
| | | EP 2467378 B1 | 27 July 2016 |
| | | ES 2599829 T3 | 03 February 2017 |
| | | HK 1170224 A1 | 22 February 2013 |
| | | IL 218669 A | 31 May 2012 |
| | | JP 2013-507355 A | 04 March 2013 |
| | | JP 5702392 B2 | 15 April 2015 |
| | | KR 10-2011-0037883 A | 13 April 2011 |
| | | MA 33880 B1 | 02 January 2013 |
| | | MX 2012003782 A | 31 August 2012 |
| | | MY 162813 A | 31 July 2017 |
| | | PE 10882012 A1 | 17 August 2012 |
| | | PE 20121088 A1 | 17 August 2012 |
| | | SG 179186 A1 | 30 May 2012 |
| | | TR 201203989 T1 | 21 September 2012 |
| | | TW 201118077 A | 01 June 2011 |
| | | TW I423962 B | 21 January 2014 |
| | | UA 110197 C2 | 10 December 2015 |
| | | US 2012-0184582 A1 | 19 July 2012 |
| | | US 8729273 B2 | 20 May 2014 |
| | | WO 2011-043568 A2 | 14 April 2011 |
| | | WO 2011-043568 A3 | 29 September 2011 |
| | | ZA 201202544 B | 29 December 2012 |
| KR 10-2009-0128488 A | 15 December 2009 | AU 2008-239017 A1 | 23 October 2008 |

Form PCT/ISA/210 (patent family annex) (July 2019)

**INTERNATIONAL SEARCH REPORT**
**Information on patent family members**

International application No.
**PCT/KR2021/015708**

| Patent document cited in search report | Publication date (day/month/year) | Patent family member(s) | Publication date (day/month/year) |
|---|---|---|---|
| | | AU 2008-239017 B2 | 27 September 2012 |
| | | BR PI0810524 A2 | 21 October 2014 |
| | | BR PI0810524 B1 | 10 September 2019 |
| | | BR PI0810524 B8 | 25 May 2021 |
| | | CA 2682391 A1 | 23 October 2008 |
| | | CA 2682391 C | 08 July 2014 |
| | | CN 101679251 A | 24 March 2010 |
| | | CN 101679251 B | 02 October 2013 |
| | | CY 1114647 T1 | 14 December 2016 |
| | | DK 2133332 T3 | 30 September 2013 |
| | | EP 2133332 A1 | 16 December 2009 |
| | | EP 2133332 A4 | 25 May 2011 |
| | | EP 2133332 B1 | 18 September 2013 |
| | | EP 2402314 A1 | 04 January 2012 |
| | | EP 2402314 B1 | 31 July 2013 |
| | | ES 2431815 T3 | 28 November 2013 |
| | | HK 1140197 A1 | 08 October 2010 |
| | | HR P20130900 T1 | 08 November 2013 |
| | | JP 5330989 B2 | 30 October 2013 |
| | | KR 10-1502957 B1 | 16 March 2015 |
| | | MX 2009010966 A | 02 November 2009 |
| | | MX 337527 B | 09 March 2016 |
| | | PL 2133332 T3 | 28 February 2014 |
| | | PT 2133332 E | 03 October 2013 |
| | | RU 2009141614 A | 20 May 2011 |
| | | RU 2477274 C2 | 10 March 2013 |
| | | SI 2133332 T1 | 28 February 2014 |
| | | SI EP2133332 T1 | 28 February 2014 |
| | | US 2010-0056521 A1 | 04 March 2010 |
| | | US 2011-0230454 A1 | 22 September 2011 |
| | | US 2013-0252955 A1 | 26 September 2013 |
| | | US 2014-0179932 A1 | 26 June 2014 |
| | | US 8003647 B2 | 23 August 2011 |
| | | US 8466152 B2 | 18 June 2013 |
| | | US 8829040 B2 | 09 September 2014 |
| | | US 8993616 B2 | 31 March 2015 |
| | | WO 2008-126898 A1 | 22 July 2010 |
| | | WO 2008-126898 A1 | 23 October 2008 |
| WO 97-27186 A1 | 31 July 1997 | AR 005549 A1 | 23 June 1999 |
| | | AU 1443297 A | 20 August 1997 |
| | | AU 715785 B2 | 10 February 2000 |
| | | BR 9707467 A | 20 July 1999 |
| | | CA 2244136 A1 | 31 July 1997 |
| | | CN 1209807 A | 03 March 1999 |
| | | CZ 9802318 A3 | 14 October 1998 |
| | | DE 19602505 A1 | 31 July 1997 |
| | | EP 0879234 A1 | 25 November 1998 |
| | | HU 9900980 A2 | 28 July 1999 |
| | | JP 2000-503972 A | 04 April 2000 |
| | | KR 10-1999-0081970 A | 15 November 1999 |

Form PCT/ISA/210 (patent family annex) (July 2019)

**INTERNATIONAL SEARCH REPORT**
**Information on patent family members**

International application No.
**PCT/KR2021/015708**

| Patent document cited in search report | Publication date (day/month/year) | Patent family member(s) | Publication date (day/month/year) |
|---|---|---|---|
| | | NO 983439 L | 25 September 1998 |
| | | PL 328228 A1 | 18 January 1999 |
| | | SK 99298 A3 | 11 January 1999 |
| | | ZA 97572 B | 04 August 1997 |
| WO 2008-126898 A1 | 23 October 2008 | AU 2008-239017 A1 | 23 October 2008 |
| | | AU 2008-239017 B2 | 27 September 2012 |
| | | BR PI0810524 A2 | 21 October 2014 |
| | | BR PI0810524 B1 | 10 September 2019 |
| | | BR PI0810524 B8 | 25 May 2021 |
| | | CA 2682391 A1 | 23 October 2008 |
| | | CA 2682391 C | 08 July 2014 |
| | | CN 101679251 A | 24 March 2010 |
| | | CN 101679251 B | 02 October 2013 |
| | | CY 1114647 T1 | 14 December 2016 |
| | | DK 2133332 T3 | 30 September 2013 |
| | | EP 2133332 A1 | 16 December 2009 |
| | | EP 2133332 A4 | 25 May 2011 |
| | | EP 2133332 B1 | 18 September 2013 |
| | | EP 2402314 A1 | 04 January 2012 |
| | | EP 2402314 B1 | 31 July 2013 |
| | | ES 2431815 T3 | 28 November 2013 |
| | | HK 1140197 A1 | 08 October 2010 |
| | | HR P20130900 T1 | 08 November 2013 |
| | | JP 5330989 B2 | 30 October 2013 |
| | | KR 10-1502957 B1 | 16 March 2015 |
| | | KR 10-2009-0128488 A | 15 December 2009 |
| | | MX 2009010966 A | 02 November 2009 |
| | | MX 337527 B | 09 March 2016 |
| | | PL 2133332 T3 | 28 February 2014 |
| | | PT 2133332 E | 03 October 2013 |
| | | RU 2009141614 A | 20 May 2011 |
| | | RU 2477274 C2 | 10 March 2013 |
| | | SI 2133332 T1 | 28 February 2014 |
| | | US 2010-0056521 A1 | 04 March 2010 |
| | | US 2011-0230454 A1 | 22 September 2011 |
| | | US 2013-0252955 A1 | 26 September 2013 |
| | | US 2014-0179932 A1 | 26 June 2014 |
| | | US 8003647 B2 | 23 August 2011 |
| | | US 8466152 B2 | 18 June 2013 |
| | | US 8829040 B2 | 09 September 2014 |
| | | US 8993616 B2 | 31 March 2015 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020200145750
- KR 1751325
- KR 1424013


### Non-patent literature cited in the description


- *Circulation Research,* 2006, vol. 98, 169-171
- *Hypertension,* 2003, vol. 41, 1183-90